## Europäisches Patentamt

## European Patent Office

## Office européen des brevets

(19)

(11) Veröffentlichungsnummer: **0 216 995**
**B1**

(12) **EUROPÄISCHE PATENTSCHRIFT**

(45) Veröffentlichungstag der Patentschrift:
**08.02.89**

(51) Int. Cl.⁴: **A61B 6/04**, A61B 6/02

(21) Anmeldenummer: 86107295.7

(22) Anmeldetag: **28.05.86**

(54) **Röntgendiagnostikanlage.**

(30) Priorität: **11.06.85 DE 3520926**

(43) Veröffentlichungstag der Anmeldung:
**08.04.87 Patentblatt 87/15**

(45) Bekanntmachung des Hinweises auf die Patenterteilung:
**08.02.89 Patentblatt 89/6**

(84) Benannte Vertragsstaaten:
**DE FR**

(56) Entgegenhaltungen:
**DE-A- 2 655 661**
**DE-A- 3 330 552**

(73) Patentinhaber: **Siemens Aktiengesellschaft Berlin und München, Wittelsbacherplatz 2, D-8000 München 2(DE)**

(72) Erfinder: **Heumann, Reiner, Dipl.-Ing. (FH), Am Mühlgarten 17, D-8521 Erlangen-Spardorf(DE)**

ACTORUM AG

## Beschreibung

Die Erfindung betrifft eine Röntgendiagnostikanlage mit einer verstellbaren Lagerstatt für ein Untersuchungsobjekt sowie einer Bildverstärker-Fernsehkette mit Bildspeicher und einem der Lagerstatt zugeordneten Meßwertaufnehmer, der ein der jeweiligen Position der Lagerstatt entsprechendes elektrisches Signal erzeugt.

Ein Gerät dieser Art ist z.B. in der DE-A 2 655 661 beschrieben. Bei einer Röntgendiagnostikanlage gemäß DE-A 3 147 128 kann die Positionierung des Untersuchungsobjektes in die gewünschte Stellung unter Durchleuchtung (Durchleuchtungskontrolle) durchgeführt werden. Der Schirm des Sichtgerätes zeigt dabei direkt die durchstrahlten Bereiche und ermöglicht dadurch die ständige Kontrolle des Positionierungsvorganges. Um die Strahlenbelastung des Untersuchungsobjektes so niedrig wie möglich zu halten, kann die Durchleuchtungskontrolle mit einer Dosisleistung durchgeführt werden, die zwar für eine Diagnose nicht mehr ausreicht, jedoch für eine Einstellung des Untersuchungsobjektes gerade noch erforderlich ist. Trotz dieser Maßnahmen ist das Untersuchungsobjekt während der Durchleuchtungskontrolle immer noch einer ständigen Strahlenbelastung ausgesetzt.

Durch die Anwendung eines Sichtgerätes mit Bildspeicher könnte eine ständige Durchleuchtung des Untersuchungsobjektes während der Positionierung vermieden werden, jedoch erfordert diese Vorgehensweise in vielen Fällen die Durchführung mehrerer Aufnahmen (Speicherschüsse) bis die gewünschte Position des Untersuchungsobjektes erreicht ist. Somit ist auch bei dieser Methode mit einer ebenfalls relativ hohen Strahlenbelastung des Patienten zu rechnen.

Der Erfindung liegt die Aufgabe zugrunde, eine Röntgendiagnostikanlage mit Bildspeicher der beschriebenen Art so auszubilden, daß eine gewünschte, hinreichend genaue Position des Untersuchungsobjektes mit Hilfe eines Speicherschusses eingestellt werden kann.

Die Aufgabe ist erfindungsgemäß dadurch gelöst, daß ein der jeweiligen Position der Lagerstatt entsprechendes elektrisches Signal einem Videomarkengenerator zugeführt ist, der im Bild auf dem Sichtgerät der Fernsehkette eine Marke an einer Stelle erzeugt, die der jeweiligen Position der Lagerstatt entspricht.

Die erfindungsgemäße Röntgendiagnostikanlage ermöglicht nach Aufnahme des Speicherschusses die Einstellung der gewünschten Position ohne weitere Strahlenbelastung des Untersuchungsobjektes.

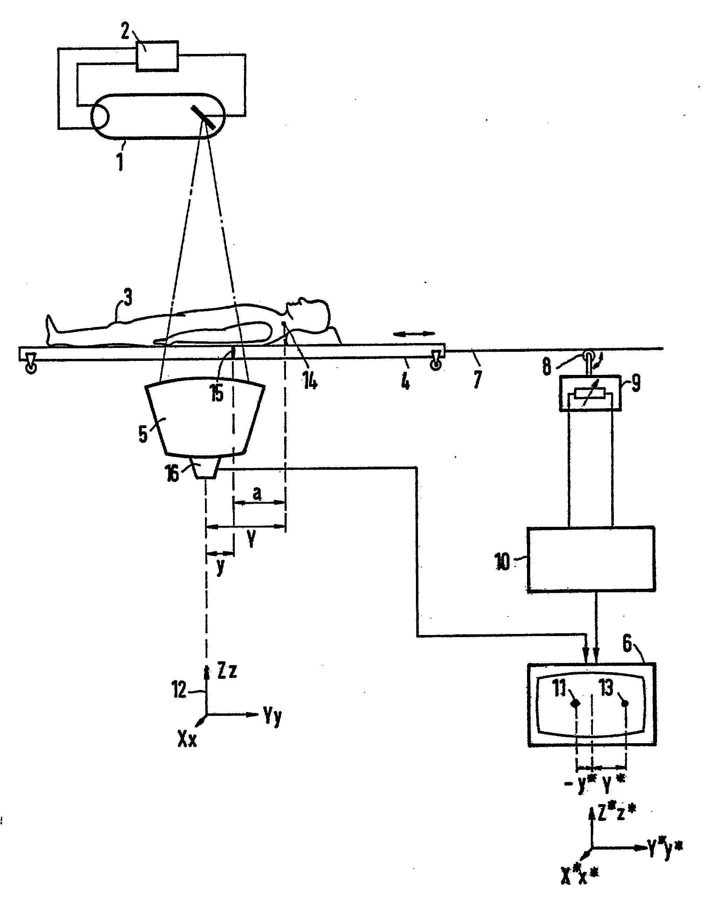

Die Erfindung ist nachfolgend anhand eines in der Zeichnung dargestellten Ausführungsbeispieles näher erläutert.

Eine Röntgenröhre 1 wird von einem Röntgengenerator 2 elektrisch gespeist. Die von ihr emittierten Röntgenstrahlen durchdringen ein Untersuchungsobjekt 3, welches auf einer für Röntgenstrahlung durchlässigen und in y-Richtung (siehe rechtshändiges Koordinatensystem 12) beweglichen Lagerstatt 4 ruht. Das auf der Schirmfläche des Bildverstärkers 5 entstehende Bild der durchstrahlten Bereiche des Patienten 3 wird in einer Fernsehkamera 16 in elektrische Signale umgesetzt, die zur bildlichen Darstellung an ein Sichtgerät 6 weitergegeben werden. Auf dem Schirm des Sichtgerätes 6 ist somit ein Bild der durchstrahlten Bereiche des Patienten 3 zu sehen.

Zusätzlich wird durch einen Videomarkengenerator 10 auf dem Schirm des Sichtgerätes 6 eine charakteristische Marke 11 erzeugt, deren Lage bezüglich der y-Richtung durch den Widerstandswert eines veränderlichen Widerstandes 9 bestimmt wird. Die Stellung des Widerstandes 9 ist über eine Rolle 8 und eine an der Lagerstatt 4 befestigte Antriebsstange 7 direkt mit der Position der Lagerstatt 4 bezüglich der y-Richtung gekoppelt. Somit wird die y-Position der Marke 11 auf dem Sichtgerät 6 durch die y-Position der Lagerstatt 4 bestimmt.

Im folgenden soll gezeigt werden, daß mit Hilfe dieser erfindungsgemäßen Anordnung eine hinreichend genaue Positionierung des Patienten bezüglich einer Raumrichtung mit nur einem Speicherschuß erreicht werden kann.

Es bezeichnen:
Y Position der zu untersuchenden Stelle des Patienten (Untersuchungspunkt 14)
$Y^*$ Position des Untersuchungs-Bildpunktes 13 auf dem Schirm
y Position der Lagerstatt
$y^*$ Position der Marke 11
$Y-y = a$ Position des Untersuchungspunktes 14 bezüglich des Lagerstattpunktes 15.
Zwischen Y und $Y^*$ sowie zwischen y und $y^*$ bestehe ein linearer Zusammenhang:

(I) $Y = c_1 . Y^*$, wobei $c_1$ = positive Proportionalitätskonstante
(II) $y = c_2 . y^*$, wobei $c_2$ = negative Proportionalitätskonstante
(III) $Y-y = a$

Man wähle $c_1$ und $c_2$ beispielsweise so, daß $c_1 = -c_2$ ist. Dies hat zur Folge, daß die Bewegung der Marke auf dem Schirm des Sichtgerätes immer entgegengesetzt zur Bewegung der Lagerstatt läuft.
Man führt nun folgendes Positionierverfahren durch:

A) Lagerstatt so positionieren, daß Marke im Zentrum des Schirmes steht, also $y^* = o$
$\rightarrow y = o$ nach (II) .

Weil Y−y = a nach (III)
→ Y = a, d.h. Untersuchungspunkt 14 ist bei a.
  B) Speicherschuß / Untersuchungs-Bildpunkt ist bei

$$Y* \stackrel{(\underline{\underline{I}})}{=} \frac{Y}{c_1} = \frac{a}{c_1}$$

  C) Marke auf gewünschten Untersuchungs-Bildpunkt des gespeicherten Bildes positionieren, d.h. die Position der Marke ist danach bei

$$y* = \frac{a}{c_1}$$

Die Positionierung der Marke bedingt eine Verschiebung der Lagerstatt in Position

$$y \stackrel{(\underline{\underline{II}})}{=} c_2 \cdot y* = c_2 \cdot \frac{a}{c_1}$$

Der Untersuchungspunkt 14 ist dann bei

$$Y \stackrel{(\underline{\underline{III}})}{=} a + y = a + c_2 \cdot \frac{a}{c_1} = a \left(1 + \frac{c_2}{c_1}\right) = 0$$

(wegen $c_1 = -c_2$) also im Zentrum des Strahlenkegels.
  D) Aufnahme / Untersuchungs-Bildpunkt ist dann bei

$$Y* = \frac{Y}{c_1} = 0 \quad ,$$

also im Zentrum des Schirmes.
  Mit Hilfe der erfindungsgemäßen Röntgendiagnostikanlage und eines durchgeführten Speicherschusses ist ohne zusätzliche Strahlenbelastung des Patienten der Untersuchungspunkt 14 in das Zentrum des Röntgenstrahlenkegels gebracht worden. Entsprechend ist der gewünschte Untersuchungs-Bildpunkt in das Zentrum des Schirmes gerückt.
  Soll der gewünschte Untersuchungs-Bildpunkt nach der Aufnahme nicht im Zentrum, sondern an einem beliebigen Punkt p des Schirmes erscheinen, so positioniert man vor Erstellung des Speicherschusses die Marke in den Punkt p.
  Eine Ausgestaltung der erfindungsgemäßen Röntgendiagnostikanlage ergibt sich durch Anwendung einer auch in die übrigen Raumrichtungen beweglichen Lagerstatt und weiterer mit ihr gekoppelter, veränderlicher Widerstände und Videomarkengeneratoren. So ist es beispielsweise möglich, bei Verwendung eines zweiten Videomarkengenerators für die x-Richtung den Patienten in der x−y-Ebene mit Hilfe nur eines Speicherschusses hinreichend genau zu positionieren.

## Patentansprüche

1. Röntgendiagnostikanlage mit einer verstellbaren Lagerstatt (4) für ein Untersuchungsobjekt (3) sowie einer Bildverstärker-Fernsehkette (5, 16, 6) mit Bildspeicher und einem der Lagerstatt (4) zugeordneten Meßwertaufnehmer (8, 9), der ein der jeweiligen Position der Lagerstatt entsprechendes elektrisches Signal erzeugt, dadurch gekennzeichnet, daß dieses Signal einem Videomarkengenerator (10) zugeführt ist, der im Bild auf dem Sichtgerät (6) der Fernsehkette (5, 16, 6) eine Marke (11) an einer Stelle erzeugt, die der jeweiligen Position der Lagerstatt (4) entspricht.

2. Röntgendiagnostikanlage nach Anspruch 1, dadurch gekennzeichnet, daß Meßwertaufnehmer (8, 9) und Videomarkengenerator (10) so ausgebildet sind, daß sich die Marke (11) im Bild proportional zur jeweiligen Positionsänderung bewegt.

**Claims**

1. X-ray diagnostic equipment having an adjustable resting-place (4) for an examination subject (3) and also an image intensifier-television chain (5, 16, 6) with image memory and a measuring sensor (8, 9) which is associated with the resting place (4) and which generates an electric signal corresponding to the respective position of the resting place, characterised in that this signal is supplied to a video marking generator (10) which generates, in the image on the display unit (6) of the television chain (5, 16, 6), a mark (11) at a point which corresponds to the respective position of the resting place (4).

2. X-ray diagnostic equipment according to claim 1, characterised in that measuring sensor (8, 9) and video marking generator (10) are developed such that the mark (11) is moved in the image proportional to the respective change in position.

**Revendications**

1. Installation de radiodiagnostic comportant un plateau formant couchette réglable (4) pour un sujet à examiner (3) ainsi qu'une chaîne de télévision à amplificateur de brillance (5, 16, 6) comportant une mémoire d'images, et un capteur de valeurs de mesure (8, 9) associé au plateau formant couchette (4) et produisant un signal électrique correspondant à la position respective du plateau formant couchette, caractérisée par le fait que ce signal est envoyé à un générateur de marques vidéo (10) qui produit, dans l'image apparaissant sur l'appareil de visualisation (6) de la chaîne de télévision (5, 16, 6), une marque (11) en un emplacement qui correspond à la position respective du plateau formant couchette (4).

2. Installation de radiodiagnostic suivant la revendication 1, caractérisée par le fait que le capteur de valeurs de mesure (8, 9) et le générateur de marques vidéo (10) sont agencés de manière que la marque (11) se déplace dans l'image proportionnellement à la variation respective de position.